(19) Europäisches Patentamt
European Patent Office
Office européen des brevets

(11) EP 0 778 827 B1

(12) EUROPÄISCHE PATENTSCHRIFT

(45) Veröffentlichungstag und Bekanntmachung des Hinweises auf die Patenterteilung:
**12.12.2001 Patentblatt 2001/50**

(21) Anmeldenummer: **95930489.0**

(22) Anmeldetag: **17.08.1995**

(51) Int Cl.7: **C07D 217/26**, C07D 263/20, C07D 263/24, C07D 209/48, C07D 413/06, C07C 271/22, C07C 229/34

(86) Internationale Anmeldenummer:
**PCT/EP95/03280**

(87) Internationale Veröffentlichungsnummer:
**WO 96/07642 (14.03.1996 Gazette 1996/12)**

# (54) VERFAHREN ZUR HERSTELLUNG EINES BETA-AMINOALKOHOLS

PROCESS FOR PRODUCING A BETA-AMINO ALCOHOL

PROCEDE DE PREPARATION D'UN BETA-AMINO-ALCOOL

(84) Benannte Vertragsstaaten:
**AT BE CH DE DK ES FR GB IE IT LI NL SE**

(30) Priorität: **03.09.1994 DE 4431530**

(43) Veröffentlichungstag der Anmeldung:
**18.06.1997 Patentblatt 1997/25**

(73) Patentinhaber: **Degussa AG**
**40474 Düsseldorf (DE)**

(72) Erfinder:
- **KOTTENHAHN, Matthias**
  **D-63579 Freigericht (DE)**
- **DRAUZ, Karlheinz**
  **D-63579 Freigericht (DE)**
- **HILPERT, Hans**
  **CH-4153 Reinach (CH)**

(56) Entgegenhaltungen:
**EP-A- 0 012 401** **EP-A- 0 635 493**

- **JOURNAL OF MEDICINAL CHEMISTRY, Bd. 20, Nr. 4, April 1977 WASHINGTON US, Seiten 510-514, RINZO NISHIZAWA ET AL 'Synthesis and structure-activity relationships of bestatin analogues,inhibitors of aminopeptidase B'**
- **JOURNAL OF MEDICINAL CHEMISTRY, Bd. 36, Nr. 2, 22.Januar 1993 WASHINGTON US, Seiten 211-219, WEI YUAN ET AL 'Development of selective tight-binding inhibitors of leukotriene A4 hydrolase'**
- **EUROPEAN JOURNAL OF MEDICINAL CHEMISTRYCHIMICA THERAPEUTICA., Bd. 26, Nr. 4, Juni 1991 PARIS FR, Seiten 435-442, A.EWENSON ET AL 'Analogues of substace P containing an alpha-hydroxy,beta-amino acid:synthesis and biological activity'**
- **BULLETIN DE LA SOCIETE CHIMIQUE DE FRANCE, Bd. 129, Nr. 6, 1992 PARIS FR, Seiten 585-593, D MELON ET AL 'Enantiomerically pure 3-amino-2-hydroxy and 5-amino-4-hydroxy acids from D-ascorbic acid'**
- **JOURNAL OF MEDICINAL CHEMISTRY, Bd. 33, Nr. 10, Oktober 1990 WASHINGTON US, Seiten 2707-2714, KINJI IIZUKA ET AL 'Orally potent human renin inhibitors derived from angiotensinogen transition state: design, synthesis, and mode of interaction'**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 778 827 B1

## Beschreibung

Die vorliegende Erfindung betrifft ein Verfahren zur Herstellung eines β-Aminoalkohols, nämlich des 2-[3(S)-Amino-2(R)-hydroxy-4-phenylbutyl]-N-tert.butyldecahydro-(4aS,8aS)-isochinolin-3(S)-carboxamids der Formel

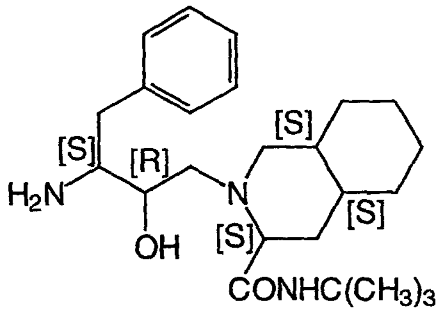

I

Die Verbindung der obigen Formel I ist beispielsweise in Beispiel 1 der Europäischen Patentpublikation 0.432.695 spezifisch beschrieben und ist ein wertvolles Zwischenprodukt zur Herstellung von pharmakologisch aktiven Verbindungen. So kann die Verbindung der Formel I wie in den Beispielen 1 und 3 der genannten Europäischen Patentpublikation beschrieben in pharmakologisch aktive Verbindungen übergeführt werden, welche sich zur Behandlung viraler Infektionen eignen, insbesondere solcher Infektionen, die durch HIV und andere Retroviren verursacht werden.

Zentraler Baustein vieler bioaktiver Wirkstoffe ist die 3-Amino-2-hydroxy-4-phenylbutancarbonsäure. Verschiedene Herstellungsverfahren finden sich deshalb in der Literatur (J.Med.Chem. 1993, 36, 211; Eur.J.Med.Chem. 1991, 26, 435; Bull.Soc.Chim.Fr. 1992, 129, 585; J.Med.Chem. 1977, 20, 510; J.Med.Chem.1990, 33, 2707). In der EP 012 401 wird die Cyanhydrinsynthesemöglichkeit an ähnlichen Verbindungen erwähnt. Die EP 635 493 beschreibt die Synthese des gleichen bioaktiven Wirkstoffs. Sie stellt mithin zwischenzeitlich veröffentlichten Stand der Technik dar.

Das erfindungsgemässe Verfahren ist dadurch gekennzeichnet, dass man

a) L-Phenylalanin der Formel

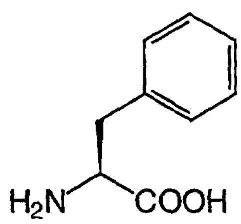

II

mit Phthalsäureanhydrid umsetzt,

b) die erhaltene 3-Phenyl-2(S)-phthalimidopropionsäure der Formel

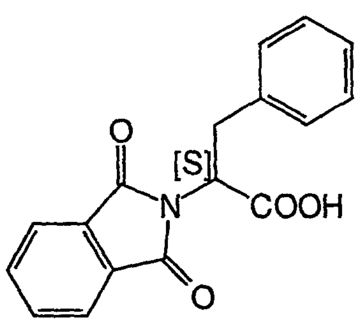

III

in das entsprechende Säurechlorid überführt,

c) das erhaltene 3-Phenyl-2(S)-phthalimidopropionsäurechlorid der Formel

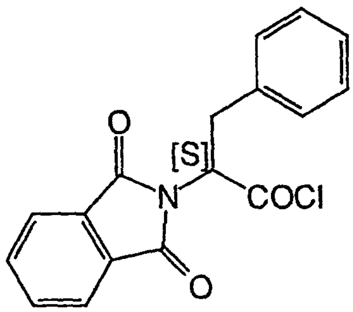

IV

reduziert,

d) das erhaltene 3-Phenyl-2(S)-phthalimidopropan-1-al der Formel

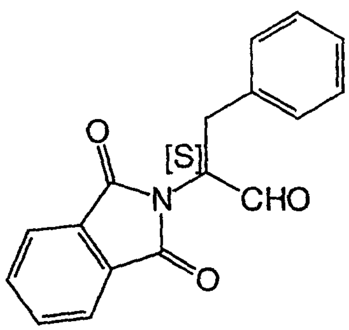

V

in das 1-Cyano-3-phenyl-2(S)-phthalimidopropan-1-ol der Formel

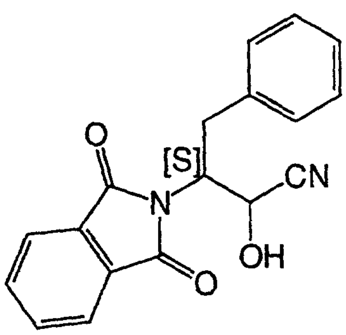

VI

umwandelt,

e) das erhaltene Nitril der Formel VI zwischen 70°C und Rückflußtemperatur mittels Mineralsäuren in Gegenwart eines Co-solvens hydrolysiert,

f) die gebildete 3(S)-Amino-2-hydroxy-4-phenylbuttersäure der Formel

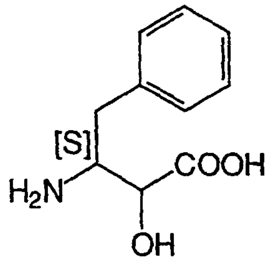

VII

mit einem ($C_1$ - $C_6$)-Alkylester oder dem Phenyl- oder Benzylester der Chlorameisensäure umsetzt,

g) eine erhaltene 3(S)-[($C_1$ - $C_6$)-Alkoxycarbonylamino-, Phenyloxycarbonylamino- oder Benzyloxycarbonylamino]-2-hydroxy-4-phenylbuttersäure der allgemeinen Formel

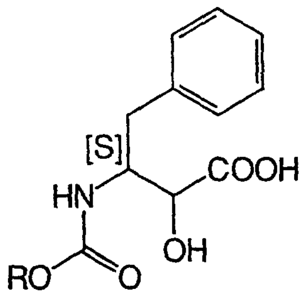

VIII

worin R ($C_1$ - $C_6$)-Alkyl, Phenyl oder Benzyl bedeutet, cyclisiert,

h) die erhaltene (4S,5S)-4-Benzyl-2-oxo-oxazolidin-5-carbonsäure der Formel

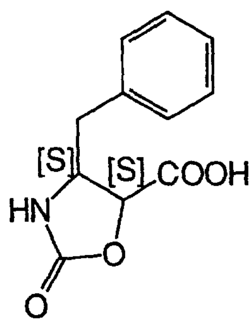

IX

mit einem ($C_1$ - $C_6$)-Alkanol verestert,

i) den so erhaltenen (4S,5S)-4-Benzyl-2-oxo-oxazolidin-5-carbonsäure-($C_1$ - $C_6$)-alkylester der allgemeinen Formel

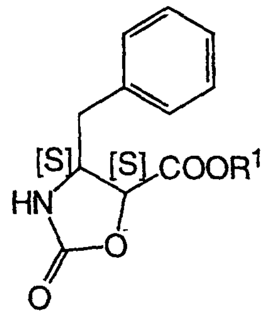

X

worin $R^1$ ($C_1$ - $C_6$)-Alkyl bedeutet,
reduziert,

j) das erhaltene (4S,5S)-4-Benzyl-5-hydroxymethyloxazolidin-2-on der Formel

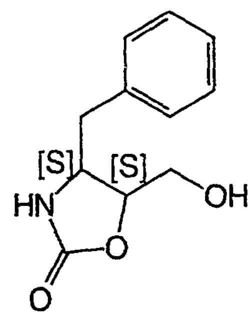

XI

in Gegenwart einer Base mit einem Sulfonsäurechlorid der allgemeinen Formel

$$R^2\text{-}SO_2Cl$$

worin $R^2$ ($C_1$ - $C_6$)-Alkyl, Phenyl oder durch Halogen, ($C_1$ - $C_6$)-Alkyl oder Nitro mono- oder di-substituiertes Phenyl bedeutet,
umsetzt,

k) einen erhaltenen Sulfonsäureester der allgemeinen Formel

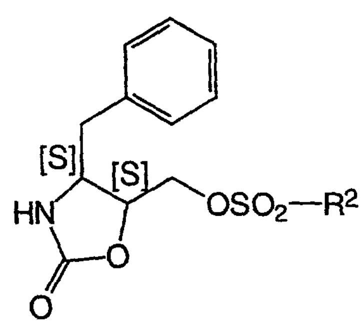

XII

worin $R^2$ die oben angegebene Bedeutung besitzt, in Gegenwart einer Base mit N-tert.Butyl-decahydro-(4aS,8aS)-isochinolin-3(S)-carboxamid der Formel

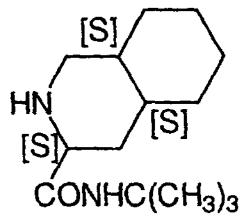

XIII

umsetzt, und

l) das entstandene 2-[(4S,5R)-4-Benzyl-2-oxo-oxazolidin-5-ylmethyl]-N-tert.butyl-decahydro-(4aS,8aS)-isochinolin-3(S)-carboxamid der Formel

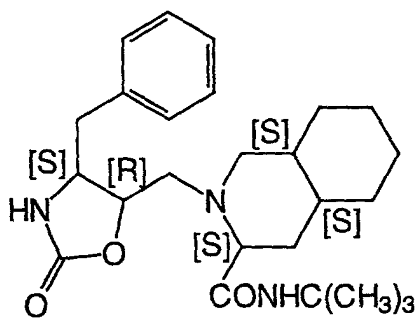

XIV

mit einer Base behandelt.

Das 3-Phenyl-2(S)-phthalimidopropan-1-al der Formel V erhält man zweckmäßig durch Erhitzen von L-Phenylalanin der Formel II mit Phthalsäureanhydrid in Toluol, Umsetzung des entstandenen N-geschützten L-Phenylalanins der Formel III mit Oxalylchlorid in Toluol und katalytischen Mengen Dimethylformamid und katalytischer (Pd/C) Hydrierung des entstandenen, dem erwünschten Aldehyd der Formel V entsprechenden Säurechlorids der Formel IV in Gegenwart eines HCl-Fängers, wie 1,2-Butylenoxid in Toluol.

Zur Herstellung des Nitrils VI wird vorteilhaft eine Lösung des Aldehyds V, z. B. in Toluol, mit wässrigem Natriumpyrosulfit versetzt, und das entstandene Additionsprodukt von Pyrosulfit und dem Aldehyd V gegebenenfalls in einem Lösungsmittel, wie Wasser oder gegebenenfalls wässrigem Methylenchlorid oder Toluol, mit Natriumcyanid behandelt. In einer Variante wird ebenfalls bevorzugt ein Gemisch des Aldehyds V und von Zink(II)bromid in einem Lösungsmittel, wie Methylenchlorid, bei -70° bis 0°C, z. B. bei -15°C, mit Trimethylsilylcyanid umgesetzt, und der entstandene Silyläther des Cyanhydrins durch Zugabe einer Lösung von Zitronensäure in Aethanol gespalten.

In einer weiteren Variante wird eine Lösung des Aldehyds V, z. B. in Toluol mit einer wässrigen NaCN-Lösung und durch Zugabe von Säure, wobei der pH-Wert zwischen 5 und 7,5 gehalten wird, zum Cyanhydrin umgesetzt.

In einer weiteren Variante wird günstigerweise eine Lösung des Aldehyds V und von Chlorameisensäurebenzylester in Methylenchlorid in Gegenwart von Benzyltriäthylammoniumchlorid unter Abkühlen, mit Natriumcyanid, zweckmässig bei -10° bis 0°C, versetzt, und das erhaltene Benzylcarbonat des Cyanhydrins in einem Lösungsmittel, wie Aethanol oder Methylenchlorid, hydriert, wobei man das Nitril VI erhält.

Die Hydrolyse des Nitrils VI erfolgt mit Vorteil mittels Mineralsäuren in Gegenwart eines Co-solvens, wie z. B. 1,4-Dioxan. Die Hydrolyse erfolgt vorzugsweise mit Salzsäure bei einer Temperatur zwischen etwa 70°C und der Rückflusstemperatur. Im Rahmen der vorliegenden Erfindung wurde gefunden, dass die Abwesenheit von 1,4-Dioxan zu einer signifikanten Reaktionsbeschleunigung führt. Der vollständige Umsatz wird nach 10 bis 20 Stunden erreicht. Durch Erhöhung der Temperatur auf >125°C (hierbei muss in einer Druckapparatur gearbeitet werden) kann die Hydrolysezeit stark gekürzt werden. Die Abtrennung der gebildeten Phthalsäure erfolgt durch Abkühlen auf 20° bis -15°C, vorzugsweise 0-5°C, und Filtrieren, wobei die Phthalsäure im Kristallisat nahezu quantitativ anfällt. Das

Filtrat enthält praktisch reine β-Aminosäure VII, die vorzugsweise ohne Isolierung in die nächste Stufe eingesetzt wird.

Die Carbamoylierung der β-Aminosäure VII wird beispielsweise durchgeführt, indem das Filtrat aus der vorhergehenden Stufe bei pH 5-12, vorzugsweise 8-10, und bei -15° bis 50°C, vorzugsweise 0-10°C, mit einem Chlorameisensäure-($C_1$ - $C_6$)-alkyl oder -benzylester versetzt wird. Die Lösung enthält praktisch reine Säure VIII, die vorzugsweise ohne Isolierung in die nächste Stufe eingesetzt wird. Gegebenenfalls kann zur Isolierung der pH-Wert nach

Ende der Reaktion (ca. 1 Stunde) mit einer Mineralsäure, vorzugsweise Salzsäure, auf 1-3 gestellt werden und die Säure VIII durch Extraktion mit Aethylacetat oder heissem Toluol und Kristallisation aus Toluol gereinigt werden.

Der Oxazolidinon-Ringschluss zur Säure IX wird zweckmässigerweise durchgeführt, indem eine wässrige Suspension der Säure VIII bei Raumtemperatur mit Natronlauge versetzt wird. Zur Isolierung der Säure IX kann das Reaktionsgemisch mit einem geeigneten organischen Lösungsmittel, wie Aethylacetat und dergleichen, extrahiert, und der Rückstand anschliessend kristallisiert werden. Zweckmässigerweise wird die Säure IX durch direkte Kristallisation aus dem Reaktionsgemisch durch Einstellen des pH-Wertes auf -1 bis +1, und Animpfen isoliert.

Die Veresterung der Säure IX wird zweckmässigerweise in Gegenwart einer katalytischen Menge einer Säure, wie Schwefelsäure, unter Erhitzen, z. B. auf Rückflusstemperatur, durchgeführt.

Die Reduktion des 2-Oxo-oxazolidin-5-carbonsäureesters X wird zweckmässigerweise in einem Lösungsmittel, wie Toluol, Tetrahydrofuran oder einem ($C_1$ - $C_6$)-Alkanol, vorzugsweise Methanol oder Aethanol, bei einer Temperatur zwischen 0° und 60°C, vorzugsweise bei 0-25°C, mittels Natrium-bis(2-methoxyäthoxy)aluminiumhydrid, Lithiumaluminiumhydrid oder vorzugsweise Natriumborhydrid durchgeführt.

Die Säure IX wird zweckmässigerweise ohne Isolierung des Esters X in den Alkohol XI übergeführt.

Die Sulfonierung des Alkohols XI wird in einem Lösungsmittel, wie Aethylacetat oder vorzugsweise Aceton oder Tetrahydrofuran, in Gegenwart einer Base, wie Triäthylamin oder vorzugsweise N-Methylmorpholin, bei einer Temperatur zwischen 0° und 60°C, vorzugsweise zwischen 20° und 40°C, durchgeführt.

Die Reaktion eines Sulfonsäureesters der Formel XII mit dem Amid XIII wird zweckmässigerweise in einem Lösungsmittel, wie Dimethylsulfoxid, einem Kohlenwasserstoff, z. B. Toluol, Triäthylamin oder einem ($C_1$ - $C_6$)--Alkanol, z. B. Aethanol, oder einem Keton, vorzugsweise 4-Methyl-2-pentanon, in Gegenwart einer Base, wie einem ($C_1$ - $C_6$)-Alkylamin oder einem Alkalimetallcarbonat, vorzugsweise Triäthylamin oder Natriumcarbonat, unter Erhitzen bis zur Rückflusstemperatur, vorzugsweise bei 50°-150°C, vorzugsweise bei 80°-110°C, durchgeführt. Zur Reinigung des entstandenen Oxazolidinons XIV wird ein leicht zu kristallisierendes Salz, wie ein Sulfonat, insbesondere das p-Toluol-, p-Bromphenyl- oder p-Nitrophenylsulfonat, durch Zugabe einer starken Säure, wie Schwefel- oder vorzugsweise Salzsäure, hergestellt.

Die Aufspaltung des Oxazolidinons XIV nach vorgängiger extraktiven Entfernung der Sulfonsäure mit einer Base, vorzugsweise Natriumbicarbonat, in einem Lösungsmittel, vorzugsweise Aethylacetat, erfolgt zweckmässigerweise in einem Lösungsmittel, wie Wasser, Aethanol oder einem Gemisch davon, mittels einer Base, wie Natrium- oder Kaliumhydroxid, unter Erhitzen bis zur Rückflusstemperatur, vorzugsweise bei 20°-100°C, vorzugsweise bei 80°C.

Die als Ausgangsstoff eingesetzte Verbindung der Formel XIII ist bekannt und entspricht derjenigen der Formel VII in der Europäischen Patentpublikation 0.432.695.

Der oben verwendete Ausdruck "($C_1$ - $C_6$)-Alkyl" betrifft geradkettige und verzweigte gesättigte Kohlenwasserstoffreste mit 1-6, vorzugsweise 1-4, Kohlenstoffatomen, wie Methyl, Aethyl, n-Propyl, Isopropyl, n-Butyl, Isobutyl, sec.Butyl, tert.Butyl, Pentyl, Hexyl und dergleichen. Der Ausdruck "Halogen" betrifft Fluor, Chlor, Brom und Jod.

Die folgenden Beispiele sollen die vorliegende Erfindung erläutern. Alle Temperaturen sind in Celsiusgraden angegeben.

<u>Beispiel 1</u> (III→IV→V)

A) Eine Suspension von 82,6 g L-Phenylalanin und 74,1 g Phthalsäureanhydrid in 600 ml Toluol wurde 8 Stunden unter Argon zum Rückfluss erhitzt. Die entstandene Suspension wurde auf Raumtemperatur abgekühlt und mit 0,5 ml Dimethylformamid, gefolgt von 66,64 g Oxalylchlorid, versetzt. Nach 2 Stunden Rühren wurde Argon in die Lösung geblasen.

B) Die das 3-Phenyl-2(S)-phthalimidopropionylchlorid enthaltende Lösung wurde mit 500 ml Toluol verdünnt und mit 72,11 g 1,2-Butylenoxid versetzt. Der Lösung wurden 23,5 g Palladium auf Kohle (5%) und 100 ml Toluol zugesetzt. Die Suspension wurde 17 Stunden unter Rühren hydriert und dann filtriert, und der Rückstand mit 200 ml Toluol gewaschen, wobei 3-Phenyl-2(S)-phthalimidopropan-1-al erhalten wurde.

<u>Beispiel 2</u> (V→VI)

Eine Suspension von 5 g 3-Phenyl-2(S)-phthalimidopropan-1-al und 4, 43 g Zinkbromid in 50 ml Methylenchlorid wurde unter Rühren bei -15° mit einer Lösung von 1,95 g Trimethylsilylcyanid in 5 ml Methylenchlorid versetzt und 5 Stunden bei -15° gerührt. Der gebildete Silyläther wurde durch Zugabe einer Lösung von 5 g Zitronensäure in 50 ml Aethanol bei -10° gespalten. Das Gemisch wurde eingeengt, und der Rückstand mit Wasser versetzt und mit

Methylenchlorid extrahiert. Die organischen Extrakte wurden getrocknet, filtriert, und das Filtrat eingeengt. Der Rückstand enthielt 5,45 g (99%) rohes 3-(1,3-Dioxo-2,3-dihydro-1H-isoindol-2-yl)-2-hydroxy-4-phenylbutyronitril als 74: 26-Gemisch der (2S, 3S)- und (2R,3S)-Isomeren, IR (KBr): 3437m (OH), 2250w (C≡N), 1775m und 1713s (C=O von Imid).

Beispiel 3 (V→VI)

Der das 3-Phenyl-2(S)-phthalimidopropan-1-al (Beispiel 1) enthaltenden Lösung wurde bei Raumtemperatur unter Rühren eine Lösung von 95,05 g Natriumpyrosulfit in 1 1 Wasser zugesetzt. Nach 4,5 Stunden Rühren wurde die das Additionsprodukt von Bisulfit und des obigen Aldehyds enthaltende wässrige Schicht mit Toluol gewaschen. Die Toluolschichten wurden mit Wasser extrahiert. Den Wasserschichten wurden 1200 ml Methylenchlorid zugesetzt, und das Gemisch wurde unter Rühren bei Raumtemperatur mit einer Lösung von 41,66 g Natriumcyanid in 330 ml Wasser versetzt. Nach 1,2 Stunden Rühren wurde Wasser zugesetzt. Die abgetrennte wässrige Schicht wurde mit Methylenchlorid extrahiert. Die organischen Schichten wurden getrocknet und filtriert, und der Rückstand wurde mit Methylenchlorid gewaschen. Die Filtrate wurden eingedampft, und der Rückstand in 200 ml Methylenchlorid gelöst. Der Lösung wurden unter Rühren bei 30° 600 ml Hexan und dann bei 0° nochmals 600 ml Hexan zugesetzt. Die Suspension wurde filtriert, und der Rückstand mit Hexan gewaschen und dann getrocknet. Man erhielt 114,02 g (74%) eines 74,7:23,5:1,4:0,4-Gemisches der Isomeren (2S,3S):(2R,3S):(2R,3R):(2S,3R) von 3-(1,3-Dioxo-2,3-dihydro-lH-isoindol-2-yl)-2-hydroxy-4-phenylbutyronitril, Smp. 127,2-130,5°, $[\alpha]_D^{20}$: -146,6° (1% in Methylenchlorid).

Beispiel 4 (V→VI)

Der das 3-Phenyl-2(S)-phthalimidopropan-1-al (Beispiel 1) enthaltenden Lösung wurde bei Raumtemperatur unter Rühren eine Lösung von 47,5 g Natriumpyrosulfit in 500 ml Wasser zugesetzt. Nach 7,5 Stunden Rühren wurde die das Additionsprodukt von Pyrosulfit und des obigen Aldehyds enthaltende wässrige Schicht mit Toluol gewaschen. Die Toluolschichten wurden mit Wasser extrahiert. Den Wasserschichten wurde unter Rühren bei Raumtemperatur eine Lösung von 24,2 g Natriumcyanid in 200 ml Wasser zugesetzt. Die Suspension wurde nach 1 Stunde Rühren filtriert, und der Rückstand mit Wasser neutral gewaschen. Nach Trocknen erhielt man 112,03 g (73%) eines 67,2: 32,8-Gemisches der Isomeren (2S,3S):(2R,3S) von 3-(1,3-Dioxo-2,3-dihydro-lH-isoindol-2-yl)-2-hydroxy-4-phenylbutyronitril,
Smp. 131-133°, $[\alpha]_D^{20}$: -150,2° (1% in Methylenchlorid).

Beispiel 5 (VI→VIII via VII)

Eine Suspension von 100 g 3-(1,3-Dioxo-2,3-dihydro-lH-isoindol-2-yl)-2-hydroxy-4-phenylbutyronitril als 73,6:26,4-Gemisch der (2S,3S)- und (2R,3S)-Isomeren (Beispiel 23) in 500 ml 25%iger Salzsäure wurde 17 Stunden zum Rückfluss erhitzt, auf 0° abgekühlt und 1 Stunde bei 0° gerührt. Nach dem Abfiltrieren der ausgefallenen Phthalsäure (52 g, 96%) wurde das Filtrat bei 25° mit 40%iger Natronlauge auf pH 7,5 gestellt und mit 46,6 ml Chlorameisensäureäthylester versetzt, wobei der pH-Wert mit 40%iger Natronlauge zwischen 7-8 gehalten wurde. Nach vollständigem Umsatz (ca. 1 Stunde) wurde die Lösung mit 37%iger Salzsäure auf pH 1 gestellt und mit Aethylacetat extrahiert. Die Extrakte wurden mit Wasser gewaschen, über Magnesiumsulfat getrocknet und filtriert, und das Filtrat eingeengt. Der Rückstand wurde aus 400 ml Toluol kristallisiert, und das Kristallisat getrocknet, wobei man 52 g (60%) eines 99:1-Gemisches der (2S,3S):(2R,3S)-Isomeren der 3-Aethoxycarbonylamino-2-hydroxy-4-phenylbuttersäure, Smp. 138,4-140,5° erhielt.
IR(KBr): 3433m und 3309s (NH,OH), 2900-2400w, br. (COOH), 1736s und 1689s (C=O).
$^1$H-NMR ($d_6$-DMSO): 12,6 (s, br., 1H, COOH); 7,30-7,00 (m, 6H, H-Ar., NH); 5,5 (s, br., 1H, OH); 4,10-3,60 (m, 4H, $CH_2CH_3$, CHN, CH-OH); 2,80-2,50 (m, 2H, $CH_2$-Ar.). MS(EI): 222 (10, M-COOH).

Beispiel 6 (VIII→IX)

Eine Suspension von 8,02 g 3-Aethoxycarbonylamino-2-hydroxy-4-phenylbuttersäure in 60 ml Wasser wurde bei Raumtemperatur mit 20 ml 3N Natronlauge versetzt und 8 1/2 Stunden bei Raumtemperatur gerührt. Die Lösung wurde mit 3,8 ml 25%iger Salzsäure auf pH 7 gestellt, unter vermindertem Druck auf 20 ml eingeengt und mit 6 ml 25%iger Salzsäure auf pH 1 gestellt. Das Reaktionsgemisch wurde danach 2 1/2 Stunden bei 0° gerührt, abfiltriert und mit Eiswasser gewaschen, wobei man 5,47 g (82%) reine (4S,5S)-4-Benzyl-2-oxo-oxazolidin-5-carbonsäure erhielt,
Smp. 177-179°.

Beispiel 7 (IX→X)

Eine Lösung von 8,85 g (4S,5S)-4-Benzyl-2-oxo-oxazolidin-5-carbonsäure in 45 ml Methanol und 0,5 ml Schwefelsäure wird 2 Stunden zum Rückfluss erhitzt, auf 15 ml eingeengt und auf -15° abgekühlt. Nach dem Abfiltrieren wird der Rückstand getrocknet, wobei man den 4-Benzyl-2-oxo-oxazolidin-5-carbonsäuremethylester als 98:2-Gemisch der (4S,5S) und (4S,5R)-Isomeren, Smp. 93-94,5°, erhält.

Beispiel 8 (X→XI)

Zu einer gerührten Lösung von 20,8 g Natriumborhydrid in 360 ml Aethanol wurde bei 15-20° während 1,5 Stunden 150 g des 98:2-Gemisches der (4S,5S)- und (4S,5R)-Isomeren des 4-Benzyl-2-oxo-oxazolidin-5-carbonsäuremethylesters gegeben und 2 Stunden nachgerührt. Die Suspension wurde bei 20° mit 540 ml Wasser versetzt, und der pH-Wert mit 165 ml 3N Salzsäure auf 7 gestellt. Die Suspension wurde 2,5 Stunden bei Raumtemperatur gerührt, 18 Stunden bei 4° stehen gelassen und dann abfiltriert. Der Rückstand wurde mit Wasser gewaschen und getrocknet und lieferte 111,6 g (84%) 99%-iges (4S,5S)-4-Benzyl-5-hydroxymethyl-oxazolidin-2-on, Smp. 167,3-168,9°, $[\alpha]_D^{20}$: -79,4° (1% in Methanol).

Beispiel 9 (XI→XII)

Zu einer Suspension von 10,4 g (4S,5S)-4-Benzyl-5-hydroxymethyl-oxazolidin-2-on in 20 ml Aceton und 6,1 ml N-Methylmorpholin wurde bei 25° eine Lösung von 4,7 ml Methansulfonylchlorid in 10 ml Aceton gegeben, und die Suspension 3 Stunden bei 25° gerührt. Es wurden erneut 1,1 ml N-Methylmorpholin zugegeben und 1 Stunde weitergerührt. Die Suspension wurde mit 80 ml halbgesättigter Natriumbicarbonat-Lösung und Aethylacetat geschüttelt, und die abgetrennte wässrige Phase mit Aethylacetat extrahiert. Die Aethylacetat-Extrakte wurden mit Wasser gewaschen, getrocknet und filtriert. Das Filtrat wurde eingedampft und lieferte 14,3 g (100%) rohen Methansulfonsäure-(4S,5S)-4-benzyl-2-oxo-oxazolidin-5-ylmethylester, DC ($SiO_2$, Aethylacetat): $R_f$ = 0,4; MS (EI): 286 $(M+H)^+$.

Beispiel 10 (XI→XII)

Analog Beispiel 9 erhielt man aus 50 g (4S,5S)-4-Benzyl-5-hydroxymethyl-oxazolidin-2-on und 80 g o-Nitrobenzolsulfonylchlorid 88,2 g (93%) o-Nitrobenzolsulfonsäure-(4S,5S)-4-benzyl-2-oxo-oxazolidin-5-ylmethylester, DC ($SiO_2$, Aethylacetat): $R_f$ = 0,38;
MS (EI): 393 $(M+H^+)$.

Beispiel 11 (XI→XII)

Zu einer gerührten Suspension von 5,55 g (4S,5S)-4-Benzyl-5-hydroxymethyl-oxazolidin-2-on in 27 ml Aceton und 6,64 g p-Toluolsulfonylchlorid wurde bei Raumtemperatur 5,6 ml Triäthylamin gegeben und 6,5 Stunden bei Raumtemperatur gerührt. Die Suspension wurde mit Wasser versetzt, bei 10° gerührt und abfiltriert, und der Rückstand mit einer Wasser/Aceton-Lösung (3:2) gewaschen und eingedampft und lieferte 9,09 g (94%) 99%-igen p-Toluolsulfonsäure-(4S,5S)-4-benzyl-2-oxo-oxazolidin-5-ylmethylester,
Smp. 148,7-150,3°, $[\alpha]_D^{20}$ : +3,0° (1% in Aceton).

Beispiel 12 (XI→XII)

Zu einer gerührten Suspension von 12,0 g (4S,5S)-4-Benzyl-5-hydroxymethyl-oxazolidin-2-on in 35 ml Tetrahydrofuran und 7,66 ml N-Methylmorpholin wurde bei Raumtemperatur in Portionen 15,4 g 4-Nitrobenzolsulfonylchlorid gegeben und 4,5 Stunden gerührt. Die Suspension wurde erneut mit 0,77 ml N-Methylmorpholin versetzt und 4,5 Stunden bei Raumtemperatur gerührt. Das Gemisch wurde mit 70 ml einer 2%-igen Natriumbicarbonat-Lösung versetzt, 1,5 Stunden gerührt und filtriert, und der Rückstand mit Wasser und Aethanol gewaschen, wobei man 21,4 g (94%) 98,5-igen p-Nitrobenzolsulfonsäure-(4S,5S)-4-benzyl-2-oxo-oxazolidin-5-ylmethylester, Smp. 148-149,5°, $[\alpha]_D^{20}$: +10.0° (1% in Aceton) erhielt.

Beispiel 13

Analog Beispiel 12 erhielt man aus 35 g (4S,5S)-4-Benzyl-5-hydroxymethyl-oxazolidin-2-on und 56,1 g p-Brombenzol-sulfonylchlorid 65,5 g (91%) p-Brombenzolsulfonsäure-(4S,5S)-4-benzyl-2-oxo-oxazolidin-5-ylmethylester,

Smp. 151,6-153°.

Beispiel 14 (V→VI)

Eine Lösung von 11,16 g 3-Phenyl-2(S)-phthalimidopropan-1-al und 0,7 g Benzyltriäthylammoniumchlorid in 70 ml Methylenchlorid wurde unter Rühren bei -10° mit 6,2 ml Chlorameisensäurebenzylester versetzt. Dann wurde eine Lösung von 3,10 g Natriumcyanid in 50 ml Wasser zugetropft. Nach 0,5 Stunden bei -10° wurde auf 0° erwärmt. Die Methylenchlorid-Phase wurde mit Wasser und mit gesättigter Kochsalz-Lösung gewaschen. Die wässrige Phase wurde mit Methylenchlorid extrahiert. Die Methylenchlorid-Extrakte wurden getrocknet und filtriert, und das Filtrat eingedampft. Der Rückstand lieferte 18,33 g eines 70:30-Gemisches von (2S,3S)- und (2R,3S)-3-(1,3-Dioxo-1,3-dihydroisoindol-2-yl)-2-benzyloxycarbonyloxy-4-phenylbutyronitril, MS (EI): 349 (6, $M^+$-$C_6H_5CH_2$), 288 (6, $M^+$-$C_6H_5CH_2OCOOH$), 91 (100, $C_6H_5CH_2$); IR (Film): 2240w (CN), 1763s und 1717s (C=O von Imid und Carbamat).

Eine Suspension von 2 g eines 70:30-Gemisches von (2S,3S)-und (2R, 3S)-3-(1,3-Dioxo-1,3-dihydroisoindol-2-yl)-2-benzyloxycarbonyloxy-4-phenylbutyronitril und 0,15 g Palladium auf Kohle (10%) in 12 ml Aethanol und 2 ml Methylenchlorid wurde 2 Stunden bei Raumtemperatur hydriert, dann filtriert und schliesslich mit Methylenchorid gewaschen, und das Filtrat eingedampft. Man erhielt auf diese Weise 1,35 g (97%) eines 72:27-Gemisches von (2S,3S)- und (2R,3S)-3-(1,3-Dioxo-1,3-dihydro-lH-isoindol-2-yl)-2-hydroxy-4-phenylbutyronitril, IR (KBr): 3450m (OH), 2250w (C≡N), 1775m und 1712s (C=O von Imid).

Beispiel 15 (XII→XIV)

A) Eine Suspension von 24,69 g p-Nitrobenzolsulfonsäure-(4S, 5S)-4-benzyl-2-oxo-oxazolidin-5-ylmethylester, 15,0 g N-tert.Butyl-decahydro-(4aS, 8aS)-isochinolin-3(S)-carboxamid und 10,0 g Natriumcarbonat in 76 ml 4-Methyl-2-pentanon wurde unter Rühren 10 Stunden zum Rückfluss erhitzt. Die Suspension wurde auf 80° abgekühlt, mit 176 ml 4-Methyl-2-pentanon und 54 ml 3N Salzsäure verdünnt, auf 40° abgekühlt und filtriert. Der Rückstand wurde mit Wasser und 4-Methyl-2-pentanon gewaschen und lieferte 35,1 g (88%) (3S,4aS,8aS)-2-[(4S,5R)-4-Benzyl-2-oxo-oxazolidin-5-ylmethyl]-N-tert.butyl-decahydro-isochinolin-3-carboxamid-p-nitrobenzolsulfonat, $[\alpha]_D^{20}$= -31,2° (1% in Dimethylformamid).

B) Man erhielt das gleiche Salz in 89% Ausbeute bei Verwendung von Triäthylamin (2 Mol-Aequivalente) anstelle von Natriumcarbonat.

Beispiel 16

Analog Beispiel 15B) erhielt man durch Umsetzen von 14 g Methansulfonsäure-(4S,5S)-4-benzyl-2-oxo-oxazolidin-5-ylmethylester mit 11,7 g N-tert.Butyl-decahydro-(4aS,8aS)-isochinolin-3(S)-carboxamid nach Zugabe von 1 Mol-Aequivalent p-Toluolsulfonsäure 11,8 g (40%) (3S,4aS,8aS)-2-[(4S,5R)-4-Benzyl-2-oxo-oxazolidin-5-ylmethyl]-N-tert.butyl-decahydro-isochinolin-3-carboxamid-p-toluolsulfonat, Smp. 203-205°.

Beispiel 17

Analog Beispiel 15B) erhielt man durch Umsetzen von 7,2 g p-Toluolsulfonsäure-(4S,5S)-4-benzyl-2-oxo-oxazolidin-5-ylmethylester mit 4,8 g N-tert.Butyl-decahydro-(4aS,8aS)-isochinolin-3(S)-carboxamid 5,4 g (45%) (3S, 4aS,8aS)-2-[(4S,5R)-4-Benzyl-2-oxo-oxazolidin-5-ylmethyl]-N-tert.butyl-decahydro-isochinolin-3-carboxamid-p-toluolsulfonat, Smp. 202-204°.

Beispiel 18

Analog Beispiel 15B) erhielt man durch Umsetzen von 30 g p-Brombenzolsulfonsäure-(4S,5S)-4-benzyl-2-oxo-oxazolidin-5-ylmethylester mit 16,8 g N-tert.Butyl-decahydro-(4aS,8aS)-isochinolin-3(S)-carboxamid 36,5 g (78%) (3S,4aS,8aS)-2-[(4S,5R)-4-Benzyl-2-oxo-oxazolidin-5-ylmethyl]-N-tert.butyl-decahydro-isochinolin-3-carboxamid-p-bromphenylsulfonat, $[\alpha]_D^{20}$= -29,9° (1% in Dimethylformamid).

Beispiel 19

Analog Beispiel 15B) erhielt man durch Umsetzen von 39,2 g 2-Nitrobenzolsulfonsäure-(4S,5S)-4-benzyl-2-oxo-oxazolidin-5-ylmethylester mit 23,8 g N-tert.Butyl-decahydro-(4aS,8aS)-isochinolin-3(S)-carboxamid nach Zugabe von 1 Mol-Aequivalent p-Toluolsulfonsäure 42 g (70%) (3S,4aS,8aS)-2-[(4S,5R)-4-Benzyl-2-oxo-oxazolidin-5-ylmethyl]-N-tert.butyl-decahydro-isochinolin-3-carboxamid-p-toluolsulfonat, $[\alpha]_D^{20}$ = -34,2° (1% in Dimethylformamid).

Beispiel 20 (XIV→I)

34,7 g (3S,4aS,8aS)-2-[(4S,5R)-4-Benzyl-2-oxo-oxazolidin-5-ylmethyl]-N-tert.butyl-decahydro-isochinolin-3-carboxamid-p-nitrobenzolsulfonat wurden zwischen 110 ml Aethylacetat und 110 ml gesättigtem Natriumbicarbonat verteilt. Die wässrige Schicht wurde mit Aethylacetat extrahiert, und die Aethylacetat-Extrakte mit 110 ml gesättigtem Natriumbicarbonat und mit Wasser gewaschen. Die organischen Extrakte wurden eingedampft, und der Rückstand mit 55 ml Aethanol verdünnt und mit einer Lösung von 11,0 g Natriumhydroxid in 55 ml Wasser unter Rühren versetzt. Das Gemisch wurde 5 Stunden zum Rückfluss erhitzt, mit 55 ml Wasser verdünnt und auf Raumtemperatur abgekühlt. Die Suspension wurde filtriert, und der Rückstand bis zu neutralem pH-Wert des Filtrats mit Wasser gewaschen. Der Rückstand wurde eingedampft und lieferte 20,7 g (93%) 2-[3(S)-Amino-2(R)-hydroxy-4-phenylbutyl]-N-tert.butyl-decahydro-(4aS,8aS)-isochinolin-3(S)-carboxamid,
Smp. 175-176°.

Beispiel 21 (III→VI via IV, V)

Eine Suspension von 100 g 3-Phenyl-2(S)-phthalimidopropionsäure in 810 ml Toluol wurde unter Rühren bei 22°C mit 0,34 ml DMF und mit 45,1 g Oxalylchlorid versetzt. Das Gemisch wurde 3 Stunden bei 22°C gerührt. Argon wurde in die Lösung durchgelassen. Dann wurde die Lösung mit 59 ml 1,2-Butylenoxyd versetzt. Dann wurde entgast und mit einer Suspension von 15,9 g Pd/C in 100 ml Toluol behandelt. Die Suspension wurde 16 Stunden unter Rühren hydriert, dann entgast und unter Argon filtriert.

Der Rückstand wurde mit Toluol gewaschen. Das Filtrat wurde unter Rühren bei 22°C mit einer Lösung von 32,2 g Natriumpyrosulfit in 320 ml Wasser versetzt. Nach 7 Stunden wurde das Gemisch mit einer Lösung von 13 g NaCN in 130 ml Wasser versetzt. Nach 30 Minuten wurde die wässrige Schicht getrennt und mit Toluol extrahiert. Die Toluolschichten wurden mit Wasser gewaschen und die vereinigten Schichten eingeengt. Die entstandene Suspension wurde unter Rühren mit Hexan verdünnt, dann filtriert. Der Rückstand wurde mit Hexan/Toluol (10/1) gewaschen. Man erhielt 80,9 g (78%) 3-(1,3-Dioxo-2,3-dihydro-1H-isoindol-2-yl)-2-hydroxy-4-phenylbutyronitril als 73,6:26,4-Gemisch der (2S,3S)- und (2R,3S)-Isomeren, Smp. 128-132°C, $[\alpha]_D^{20}$: -151,4° (1% in Methylenchlorid).

Beispiel 22 (VI→IX via VII, VIII)

Eine Suspension von 300 g 3-(1,3-Dioxo-2,3-dihydro-1H-isoindol-2-yl)- 2-hydroxy-4-phenylbutyronitril als 74:26-Gemisch der (2S,3S)- und (2R,3S)-Isomeren (Beispiel 2) in 1500 ml 25%iger Salzsäure wurde 17 Stunden zum Rückfluss erhitzt, mit Aktivkohle behandelt, auf 0°C abgekühlt und 1 Stunde gerührt. Nach dem Filtrieren der Suspension wurde das Filtrat bei 0-20°C mit 40%iger Natronlauge auf pH 8,5 gestellt und mit 121 ml Chlorameisensäuremethylester versetzt, wobei der pH-Wert mit 40%iger Natronlauge zwischen 8-9 gehalten wurde. Nach Rühren bei 0°C wurde die Lösung bei 22°C mit 98 ml 40%iger Natronlauge auf pH 12,9 gestellt. Nach 4 Stunden wurde die Lösung mit 160 ml 25%iger Salzsäure auf pH 3,5 gestellt und mit weiteren 80 ml 25%iger Salzsäure versetzt. Das Gemisch wurde mit (4S,5S)-4-Benzyl-2-oxo-oxazolidin-5-carbonsäure geimpft und mit 25%iger Salzsäure auf pH -0,4 gestellt. Nach 2 Stunden bei -10°C wurde die Suspension filtriert. Man erhielt 131,6 g (61%) eines 97,2:2,8-Gemisches der (4S,5S):(4S,5R)-Isomeren der 4-Benzyl-2-oxo-oxazolidin-5-carbonsäure, Smp. 172-174°C, $[\alpha]_D^{20}$: -106,2° (1% in Methanol).

Beispiel 23 (IX→XI via X)

Eine Lösung von 50 g eines 97,2:2,8-Gemisches der (4S,5S) : (4S,5R)-Isomeren der 4-Benzyl-2-oxo-oxazolidin-5-carbonsäure in 300 ml Methanol wurde mit 1 ml Schwefelsäure versetzt und 3,5 Stunden zum Rückfluss erhitzt. Die Lösung wurde auf 0°C abgekühlt und mit einer Lösung von 30% Natriummethoxid in Methanol auf pH 7 gestellt. Der Lösung wurden bei 0-3°C 10,26 g Natriumborhydrid zugesetzt. Nach 1 Stunde Rühren bei 0°C wird die Suspension bei 0-20°C mit 25%iger Salzsäure auf pH 1 gestellt. Nach Zusatz von Wasser und Rühren bei -10°C wird die Suspension filtriert. Man erhält 41,5 g (89%) 99%iges (4S,5S)-4-Benzyl-5-hydroxymethyl-oxazolidin-2-on, Smp. 168-171°C, $[\alpha]_D^{20}$: -79,1° (1% in Methanol).

Beispiel 24 (IX→XI)

a) Einer Lösung von 22,1 g eines 97,2:2,8-Gemisches der (4S,5S):(4S,5R)-Isomeren der 4-Benzyl-2-oxo-oxazolidin-5-carbonsäure (Beispiel 22) in 120 ml Ethanol werden 1,2 ml Schwefelsäure (95-97%ig) zugesetzt und es wird 2 Stunden unter Rückfluss gekocht. Danach wird die Lösung durch destillative Abnahme von 90 ml Ethanol konzentriert.

b) Die Lösung von a) wird bei max. 25°C einer Suspension von 5,3 g Natriumborhydrid in 40 ml Ethanol zugetropft. Nach beendeter Zugabe wird 2-3 Stunden nachgerührt. Zur weissen Suspension wird Wasser zugesetzt und mit konz. HCl ein pH-Wert von 7 eingestellt. Es wird zunächst 2 Stunden bei 22°C, dann weitere 2 Stunden bei 0°C nachgerührt. Der Feststoff wird abfiltriert und mit Eiswasser gewaschen.

Anschliessend wird im Vakuum bei 45°C getrocknet. Ausbeute: 18,68 g (90,1%) eines 98:2-Gemisches des (4S,5S)(4S,5R)-Isomeren des 4-Benzyl-5-hydroxymethyloxazolidin-2-ons.

Beispiel 25 (III→IX, via IV, V, VI, VII, VIII)

Zu einer gerührten Suspension von 118,1 g Phthaloyl-L-phenylalanin in 720 ml Toluol wurden 0,4 ml Dimethylformamid zugegeben und innerhalb von 30 Minuten 37,7 ml Oxalylchlorid zugetropft. Nach Ende der Gasentwicklung wurde 1 Stunde bei Raumtemperatur nachgerührt und dann im Vakuum 20 Minuten im Vakuum eingeengt. Anschliessend wurden 95 ml 1,2-Epoxybutan zugesetzt und 20 Minuten bei Raumtemperatur gerührt. Es wurden 18,8 g Palladium auf Kohle (5%) zugesetzt und bei 4 bar Wasserstoff-Ueberdruck bis zum Ende der Wasserstoffaufnahme hydriert. Der Katalysator wurde abfiltriert und der Rückstand mit 70 ml Toluol gewaschen, wobei 3-Phenyl-2(S)-phthalimidopropan-1-al in toluolischer Lösung erhalten wurde.

Diese Lösung wurde mit 100 ml Wasser versetzt und unter Rühren wurde innerhalb von einer Stunde bei 20-25°C eine Lösung von 20,7 g NaCN in 93 ml Wasser zugetropft. Durch gleichzeitiges Zutropfen von halbkonz. HCl wurde der pH-Wert zwischen 6 und 7,2 gehalten. Nach Ende der Zugabe wurde 10 Stunden bei Raumtemperatur nachgerührt, die Phasen getrennt, wobei die wässrige Phase mit 100 ml Toluol nachextrahiert wurde und die vereinigten Toluolphasen mit 100 ml Wasser bei pH 3 ausgewaschen. Nach der Phasentrennung wurde ein 74,5:25,5-Gemisch der Isomeren (2S,3S):(2R,3S) von 3-(1,3-Dioxo-2,3-dihydro-1H-isoindol-2-yl)-2-hydroxy-4-phenylbutyronitril in toluolischer Lösung erhalten.

Nach Zusatz von 100 ml 21%iger wässriger HCl wurde das Toluol azeotrop abdestilliert, wobei das wässrige Destillat in den Sumpf zurückgeführt wurde. Nach Abdestillieren des Toluols wurden weitere 415 ml 21%ige wässrige HCl zugesetzt und für 16 Stunden unter Rückfluss gehalten. Nun wurden 463 g wässrige HCl abdestilliert und der Sumpf mit 165 ml Wasser verdünnt. Nach Abkühlen auf 10°C wurde der Feststoff abfiltriert und zweimal mit je 32 ml Wasser gewaschen.

70% der wässrigen Filtrate (284,4 g) (sie enthalten die 3-Amino-2-hydroxy-4-phenylbuttersäure) wurden nun mit 70 ml Isobutyl-methylketon extrahiert und anschliessend die wässrige Produktphase mit 141 ml Wasser verdünnt. Nach Zusatz von 27 ml NaOH 40%ig bei 20-25°C wurde auf <5°C abgekühlt und 35 ml Methylchlorformiat zugetropft. Währenddessen wurde durch parallele Zugabe von 57 ml NaOH 40%ig der pH-Wert im Bereich von 8-9 gehalten. Nach einer halben Stunde Nachreaktion wurde die Lösung mit 40,4 ml NaOH 40%ig versetzt, wobei ein pH-Wert von 13,3 erhalten wurde. Nach einer Stunde wurde mit 61 ml wässriger HCl 37%ig ein pH-Wert von 3,3 eingestellt und die wässrige Phase mit 70 ml Isobutyl-methylketon extrahiert. Danach wurde auf 60°C erhitzt und weitere 42,6 ml HCl 37%ig zugesetzt. Beim Abkühlen auf 0°C fiel ein weisser kristalliner Niederschlag aus, der nach einer Stunde Rühren bei 0°C abfiltriert und zweimal mit 100 ml Eiswasser gewaschen wurde.

Das Feuchtprodukt wurde in 230 ml Methyl-tert.butylether aufgeschlämmt und bei Normaldruck azeotrop entwässert. Dann wurde auf 0°C abgekühlt, abfiltriert, zweimal mit 20 ml Methyl-tert.butylether gewaschen und getrocknet. Ausbeute 27,72 g (45% der Theorie bez. auf eingesetztes Phthaloyl-L-phenylalanin) 4-Benzyl-2-oxo-oxazolidin-5-carbonsäure. Diastereomerenverhältnis 4S,5S:4S,5R = 96,3:3,7.

## Patentansprüche

1. Verfahren zur Herstellung von 2-[3(S)-Amino-2(R)-hydroxy-4-phenylbutyl]-N-tert butyl-decahydro-(4aS,8aS)-isochinolin-3(S)-carboxamid der Formel

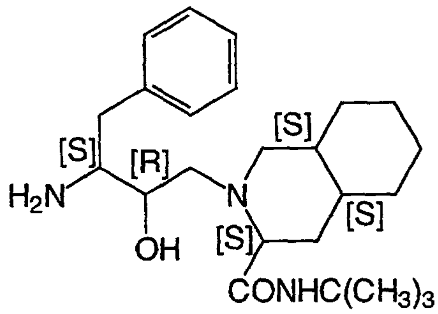

I

**dadurch gekennzeichnet, dass** man

a) L-Phenylalanin der Formel

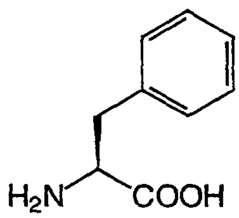

II

mit Phthalsäureanhydrid umsetzt,

b) die erhaltene 3-Phenyl-2(S)-phthalimidopropionsäure der Formel

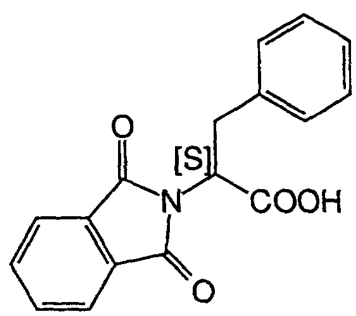

III

in das entsprechende Säurechlorid überführt,

c) das erhaltene 3-Phenyl-2(S)-phthalimidopropionsäurechlorid der Formel

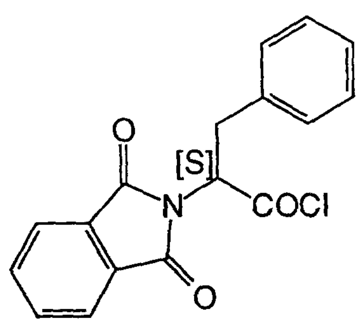

IV

reduziert,

d) das erhaltene 3-Phenyl-2(S)-phthalimidopropan-1-al der Formel

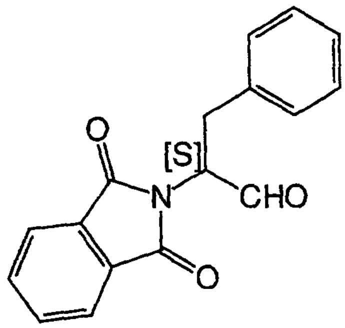

V

in das 1-Cyano-3-phenyl-2(S)-phthalimidopropan-1-ol der Formel

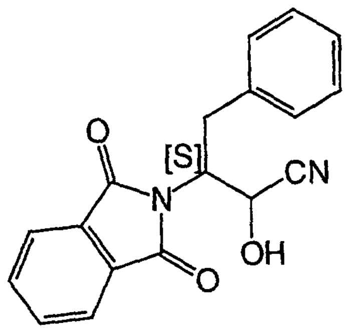

VI

umwandelt,

e) das erhaltene Nitril der Formel VI zwischen 70°C und Rückflußtemperatur mittels Mineralsäuren in Gegenwart eines Co-solvens, hydrolysiert,

f) die gebildete 3(S)-Amino-2-hydroxy-4-phenylbuttersäure der Formel

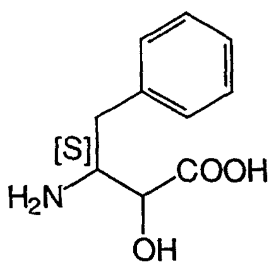

VII

mit einem ($C_1$ - $C_6$)-Alkylester oder dem Phenyl- oder Benzylester der Chlorameisensäure umsetzt,

g) eine erhaltene 3(S)-[($C_1$ - $C_6$)-Alkoxycarbonylamino-, Phenyloxycarbonylamino- oder Benzyloxycarbonylamino]-2-hydroxy-4-phenylbuttersäure der allgemeinen Formel

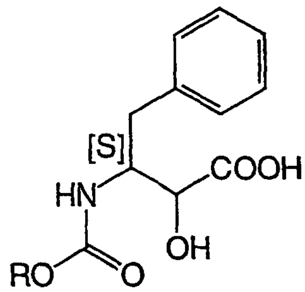

VIII

worin R ($C_1$ - $C_6$)-Alkyl, Phenyl oder Benzyl bedeutet, cyclisiert,

h) die erhaltene (4S,5S)-4-Benzyl-2-oxo-oxazolidin-5-carbonsäure der Formel

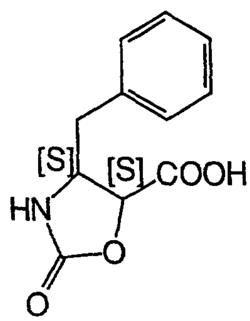

IX

mit einem ($C_1$ - $C_6$)-Alkanol verestert,

i) den so erhaltenen (4S,5S)-4-Benzyl-2-oxo-oxazolidin-5-carbonsäure-($C_1$ - $C_6$) -alkylester der allgemeinen Formel

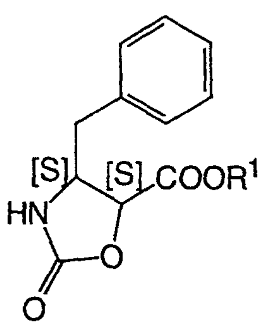

X

worin $R^1$ ($C_1$ - $C_6$)-Alkyl bedeutet, reduziert,

j) das erhaltene (4S,5S)-4-Benzyl-5-hydroxymethyloxazolidin-2-on der Formel

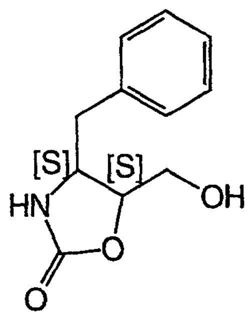

XI

in Gegenwart einer Base mit einem Sulfonsäurechlorid der allgemeinen Formel

$$R^2\text{-}SO_2Cl$$

worin $R^2$ $(C_1 - C_6)$-Alkyl, Phenyl oder durch Halogen, $(C_1 - C_6)$-Alkyl oder Nitro mono- oder di-substituiertes Phenyl bedeutet,
umsetzt,

k) einen erhaltenen Sulfonsäureester der allgemeinen Formel

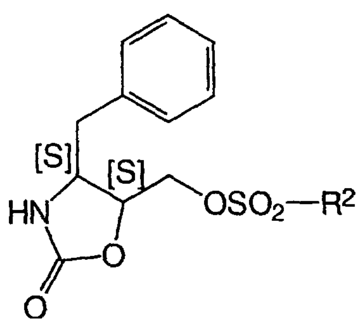

XII

worin $R^2$ die oben angegebene Bedeutung besitzt, in Gegenwart einer Base mit N-tert.Butyl-decahydro-(4aS, 8aS)-isochinolin-3(S)-carboxamid der Formel

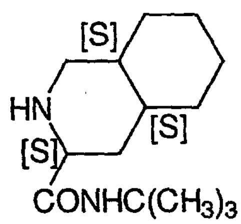

XIII

umsetzt, und

l) das entstandene 2-[(4S,5R)-4-Benzyl-2-oxo-oxazolidin-5-ylmethyl]-N-tert.butyl-decahydro-(4aS,8aS)-isochinolin-3(S)-carboxamid der Formel

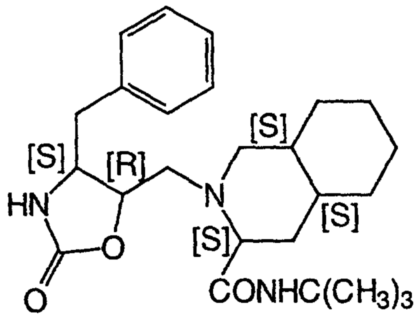

XIV

mit einer Base behandelt.

**2.** Verfahren zur Herstellung einer 3(S)-[($C_1$ - $C_6$)-Alkoxycarbonylamino-, Phenoxycarbonylamino- oder Benzyloxycarbonylamino]-2-hydroxy-4-phenylbuttersäure der allgemeinen Formel

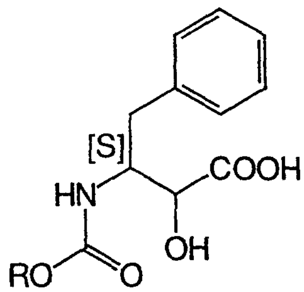

VIII

worin R ($C_1$ - $C_6$)-Alkyl, Phenyl oder Benzyl bedeutet,
**dadurch gekennzeichnet, dass** man

a) das 1-Cyano-3-phenyl-2(S)-phthalimidopropan-1-ol der Formel

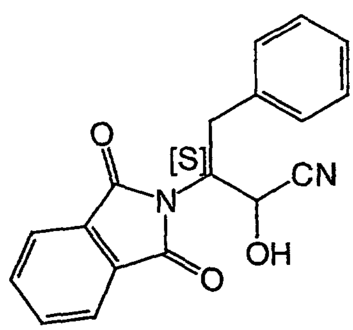

VI

zwischen 70°C und Rückflußtemperatur mittels Mineralsäuren in Gegenwart eines Co-solvens, hydrolysiert und

b) die gebildete 3(S)-Amino-2-hydroxy-4-phenylbuttersäure der Formel

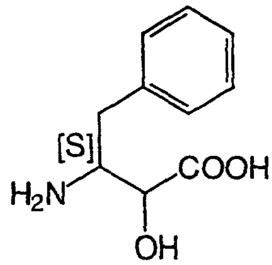

VII

mit einem ($C_1$ - $C_6$)-Alkylester oder dem Phenyl- oder Benzylester der Chlorameisensäure umsetzt.

**3.** Verfahren zur Herstellung der (4S,5S)-4-Benzyl-2-oxo-oxazolidin-5-carbonsäure der Formel

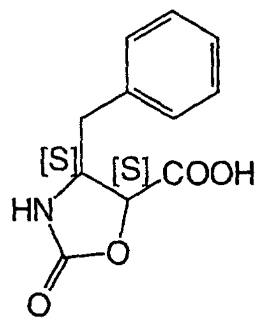

IX

nach Anspruch 1, **dadurch gekennzeichnet, dass** man ausgehend von Verbindung III die Schritte b) bis h) ohne Isolierung der Zwischenprodukte durchführt und in Schritt e) die Hydrolyse in salzsaurem Medium vornimmt.

**Claims**

**1.** Process for the production of 2-[3(S)-amino-2(R)-hydroxy-4-phenylbutyl)-N-tert.-butyldecahydro-(4aS,8aS)-isoquinoline-3(S)-carboxamide of the formula

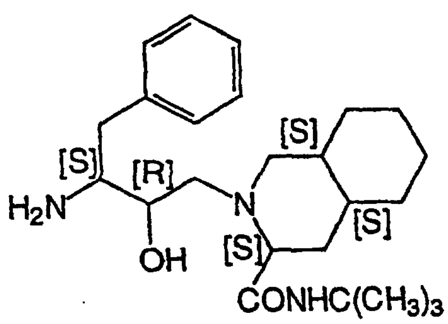

I

**characterised in that**

a) L-phenylalanine of the formula

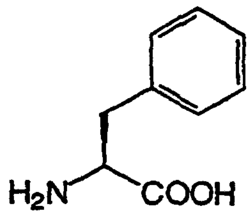

II

is reacted with phthalic anhydride,

b) the resultant 3-phenyl-2(S)-phthalimidopropionic acid of the formula

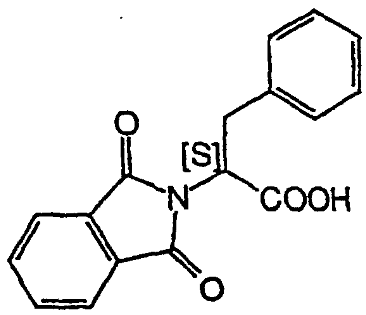

III

is converted into the corresponding acid chloride,

c) the resultant 3-phenyl-2(S)-phthalimidopropionic acid chloride of the formula

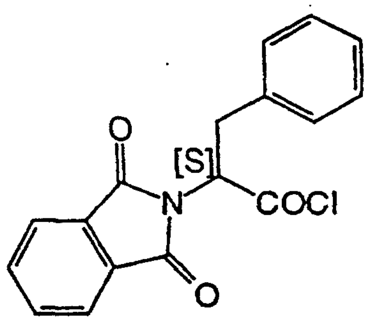

IV

is reduced,

d) the resultant 3-phenyl-2(S)-phthalimidopropan-1-al of the formula

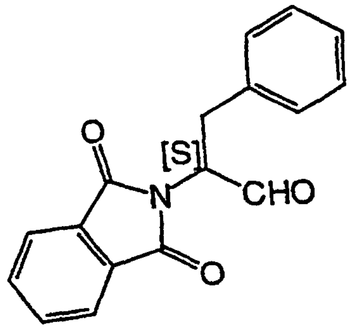

V

is converted into the 1-cyano-3-phenyl-2(S)-phthalimidopropan-1-ol of the formula

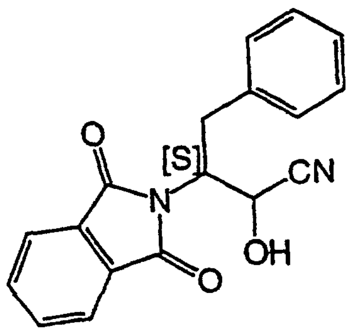

VI

e) the resultant nitrile of the formula VI is hydrolysed by means of mineral acids in the presence of a cosolvent at between 70°C and reflux temperature,

f) the 3(S)-amino-2-hydroxy-4-phenylbutyric acid formed of the formula

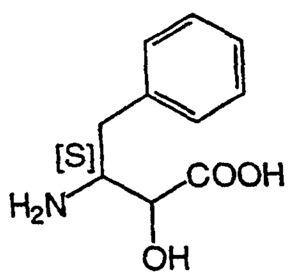

VII

is reacted with a ($C_1$-$C_6$) alkyl ester or the phenyl or benzyl ester of chloroformic acid,

g) a resultant 3(S)-[($C_1$-$C_6$)-alkoxycarbonylamino-, phenyloxycarbonylamino- or benzyloxycarbonylamino]-2-hydroxy-4-phenylbutyric acid of the general formula

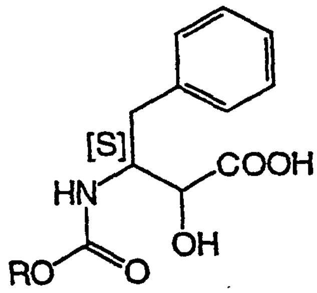

VIII

in which R means ($C_1$-$C_6$) alkyl, phenyl or benzyl, is cyclised,

h) the resultant (4S,5S)-4-benzyl-2-oxo-oxazolidine-5-carboxylic acid of the formula

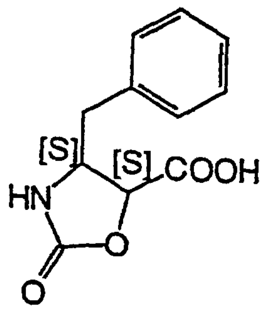

IX

is esterified with a ($C_1$-$C_6$) alkanol,

i) the resultant (4S,5S)-4-benzyl-2-oxo-oxazolidine-5-carboxylic acid ($C_1$-$C_6$) alkyl ester of the general formula

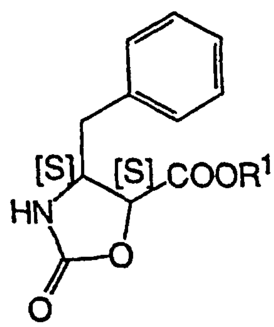

X

in which $R^1$ means ($C_1$-$C_6$) alkyl, is reduced,

j) the resultant (4S,5S)-4-benzyl-5-hydroxymethyloxazolidin-2-one of the formula

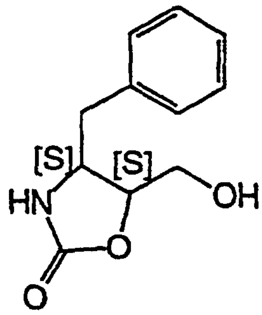

XI

is reacted in the presence of a base with a sulfonic acid chloride of the general formula

$$R^2\text{-}SO_2Cl$$

in which $R^2$ means ($C_1$-$C_6$) alkyl, phenyl or phenyl mono-or disubstituted by ($C_1$-$C_6$) alkyl or nitro,

k) a resultant sulfonic acid ester of the general formula

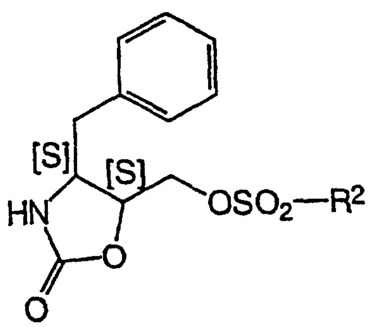

XII

in which $R^2$ has the above-stated meaning, is reacted in the presence of a base with N-tert.-butyldecahydro-(4aS,8aS)-isoquinoline-3(S)-carboxamide of the formula

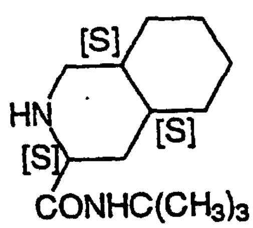

XIII

and

l) the resultant 2-[(4S,5R)-4-benzyl-2-oxo-oxazolidin-5-ylmethyl]-N-tert.-butyldecahydro-(4aS,8aS)-isoquinoline-3(S)-carboxamide of the formula

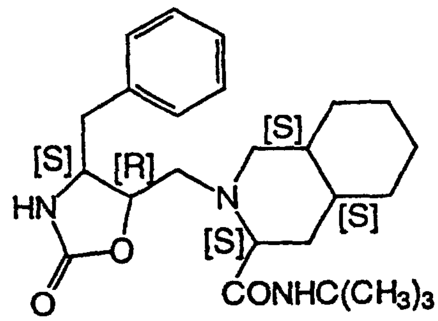

XIV

is treated with a base.

**2.** Process for the production of a 3(S)-[($C_1$-$C_6$)-alkoxycarbonylamino-, phenoxycarbonylamino- or benzyloxycarbonylamino]-2-hydroxy-4-phenylbutyric acid of the general formula

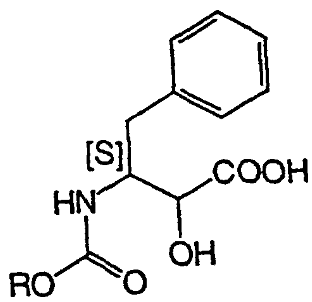

VIII

in which R means ($C_1$-$C_6$) alkyl, phenyl or benzyl, **characterised in that**

a) the 1-cyano-3-phenyl-2(S)-phthalimidopropan-1-ol of the formula

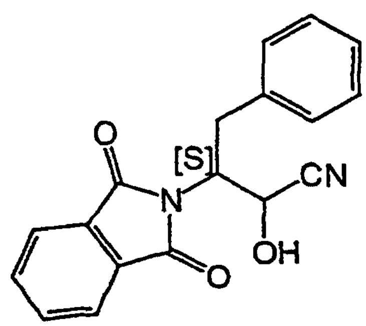

VI

is hydrolysed by means of mineral acids in the presence of a cosolvent at between 70°C and reflux temperature and

b) the 3(S)-amino-2-hydroxy-4-phenylbutyric acid formed of the formula

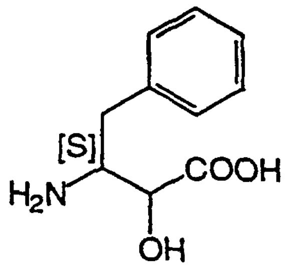

VII

is reacted with a ($C_1$-$C_6$) alkyl ester or the phenyl or benzyl ester of chloroformic acid.

**3.** Process for the production of the (4S,5S)-4-benzyl-2-oxo-oxazolidine-5-carboxylic acid of the formula

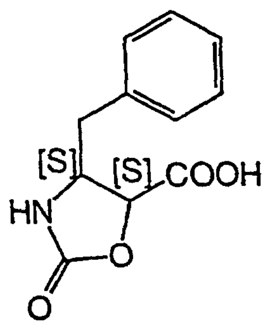

IX

according to claim 1, **characterised in that**, starting from compound III, steps b) to h) are performed without isolating the intermediate products and the hydrolysis in step e) is performed in a hydrochloric medium.

## Revendications

**1.** Procédé pour la préparation de 2-[3(S)-amino-2(R)-hydroxy-4-phénylbutyl]-N-tert-butyl-décahydro-(4aS, 8aS)-isoquinoléine-3(S)-carboxamide de formule

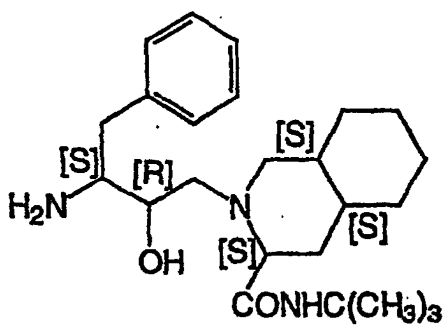

I

**caractérisé**

a) **en ce qu'**on transforme de la L-phénylalanine de formule

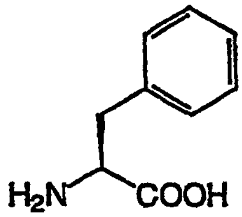

II

avec de l'anhydride de l'acide phtalique,

b) **en ce qu'**on transforme l'acide 3-phényl-2(S)-phtalunidopropionique obtenu de formule

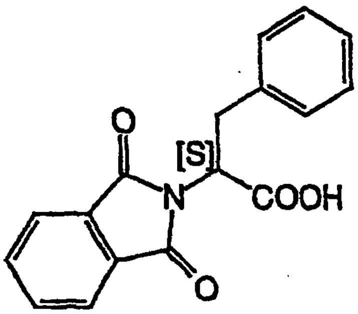

III

en chlorure d'acide correspondant,

c) **en ce qu'**on réduit le chlorure de l'acide 3-phényl-2(S)-phtalimidopropionique obtenu de formule

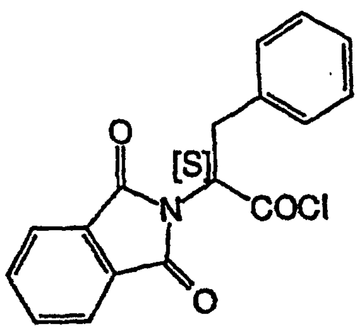

IV

d) **en ce qu'**on transforme le 3-phényl-2(S)-phtalimidopropan-l-al obtenu de formule

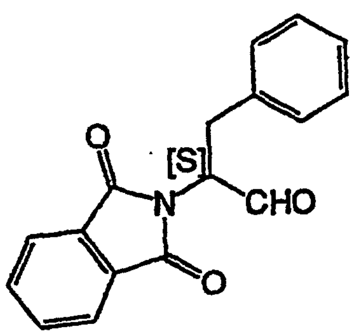

V

en 1-cyano-3-phényl-2(S)-phtalimidopropan-1-ol de formule

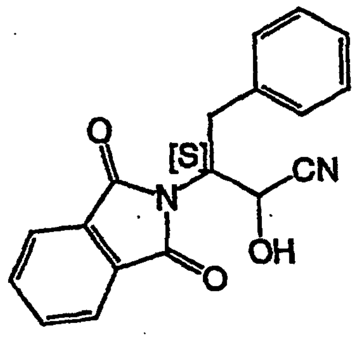

VI

e) **en ce qu'**on hydrolyse le nitrile obtenu de formule VI entre 70 °C et la température de reflux au moyen d'acides minéraux en présence d'un cosolvant,
f) **en ce qu'**on fait réagir l'acide 3(S)-amino-2-hydroxy-4-phénylbutyrique de formule

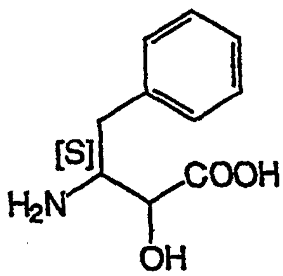

VII

avec un ester d'alkyle en $C_1$ à $C_4$ ou l'ester phénylique ou l'ester benzylique de l'acide chloroformique,
g) **en ce qu'**on cyclise un acide 3(S)-[(alkoxy en $C_1$ à $C_6$)-carbonylamino]-2-hydroxy-4-phénylbutyrique, un acide 3(S)-(phényloxycarbonylanuno]-2-hydroxy-4-phénylbutyrique ou un acide 3(S)-[benzyloxycarbonylamino]-2-hydroxy-4-phénylbutyrique de formule générale

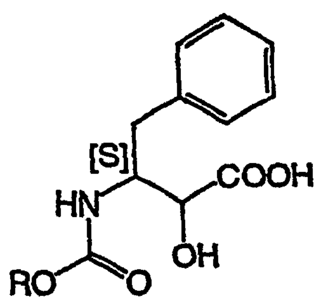

VIII

dans laquelle R signifie un groupe alkyle en $C_1$ à $C_6$, un groupe phényle ou un groupe benzyle,
h) **en ce qu'**on estérifie l'acide (4S,5S)-4-benzyl-2-oxo-oxazolidine-5-carboxylique obtenu de formule

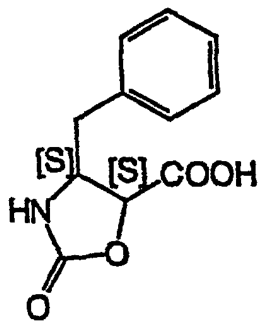

IX

avec un alcanol en $C_1$ à $C_6$,

i) en ce qu'on réduit l'ester alkylique en $C_1$ à $C_6$ de l'acide (4S,5S)-4-benzyl-2-oxo-oxazolidine-5-carboxylique ainsi obtenu de formule générale

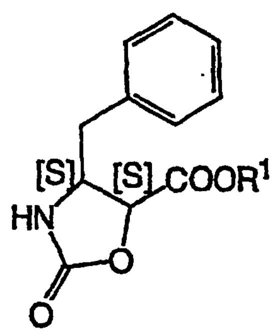

X

dans laquelle $R^1$ signifie un groupe alkyle en $C_1$ à $C_6$

j) en ce qu'on fait réagir la (4S,5S)-4-benzyl-5-hydroxyméthyl-oxazolidin-2-one de formule

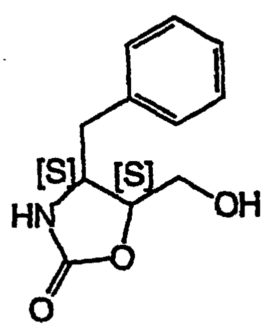

XI

en présence d'une base avec un chlorure d'acide sulfonique de formule générale

$$R^2\text{-}SO_{Cl}$$

dans laquelle $R^2$ signifie un groupe alkyle en $C_1$ à $C_6$, un groupe phényle ou un groupe phényle monosubstitué ou disubstitué par un halogène, un groupe alkyle en $C_1$ à $C_6$ ou un groupe nitro,

k) en ce qu'on fait réagit un ester de l'acide sulfonique obtenu de formule générale

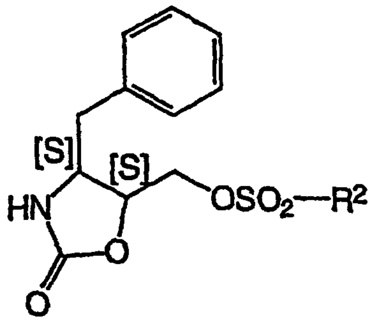

XII

dans laquelle $R^2$ présente la signification susmentionnée, en présence d'une base, avec du N-tert-butyl-décahydro-(4aS,8aS)-isoquinoléine-3(S)-carboxamide de formule

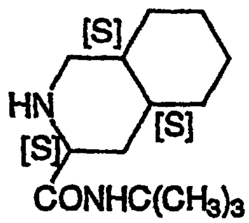

XIII

et

l) en ce qu'on traite le 2-[(4S,5R)-4-benzyl-2-oxo-oxazolidin-5-ylméthyl]-N-tert-butyl-décahydro-(4aS, 8aS)-isoquinoléine-3(S)-carboxamide de formule

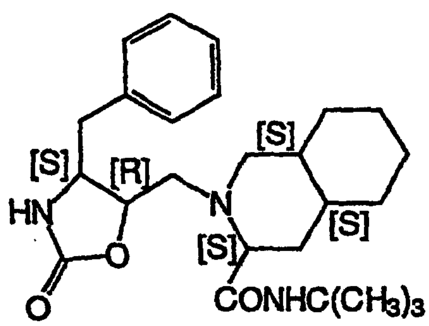

XIV

avec une base.

**2.** Procédé pour la préparation d'un acide 3(S)-[(alkoxy en $C_1$ à $C_6$)-carbonylamino]-2-hydroxy-4-phénylbutyrique, d'un acide 3(S)-[phénoxycarbonylamino]-2-hydroxy-4-phénylbutyrique ou d'un acide 3(S)-[benzyloxycarbonylamino]-2-hydroxy-4-phénylbutyrique de formule générale

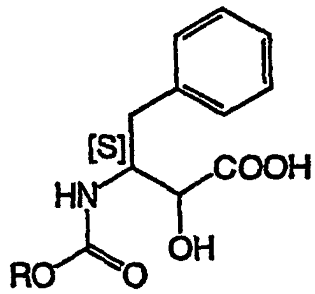

VIII

dans laquelle R signifie un groupe alkyle en $C_1$ à $C_6$, un groupe phényle ou un groupe benzyle, **caractérisé**

a) **en ce qu'**on hydrolyse le 1-cyano-3-phényl-2(S)-phtalimidopropan-1-ol de formule

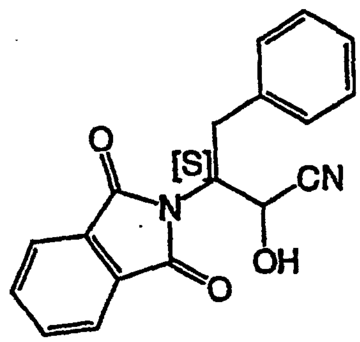

VI

entre 70 °C et la température de reflux au moyen d'acides minéraux en présence d'un cosolvant et
b) **en ce qu'**on fait réagir l'acide 3(S)-amino-2-hydroxy-4-phénylbutyrique de formule

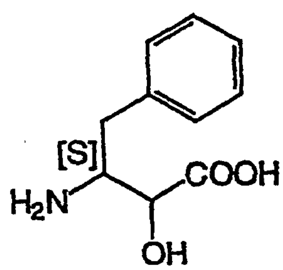

VII

avec un ester alkylique en $C_1$ à $C_6$ ou l'ester phénylique ou l'ester benzylique de l'acide chloroformique.

**3.** Procédé pour la préparation de l'acide (4S,5S)-4-benzyl-2-oxo-oxazoline-5-carboxylique de formule

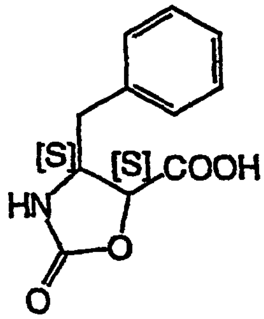

IX

selon la revendication 1, **caractérisé en ce qu'**on réalise les étapes b) et h) partant du composé III sans isolement des produits intermédiaires et **en ce qu'**on réalise l'hydrolyse dans l'étape e) en milieu acide.